# EUROPEAN PATENT APPLICATION

(11) **EP 0 937 779 A2**
(43) Date of publication of application: **25.08.1999**
(21) Application number: 99102683.2
(22) Date of filing: 12.02.1999
(51) Int. Cl.: C12Q 1/48, C12Q 1/527, C12Q 1/32

(54) **Cell damage marker**

(30) Priority: 13.02.1998 JP 4634998
(71) Applicant: ORIENTAL YEAST CO., LTD., Tokyo (JP)
(72) Inventor: Yamori, Yukio, Kyoto-shi, Kyoto-fu (JP); Ikeda, Katsumi c/o Hagisaki-manshion 303, Kyoto-shi, Kyoto-fu (JP); Uchida, Kohji, Hikone-shi, Shiga-ken (JP); Taniguchi, Yoshiyuki, Kawaguchi-shi, Saitama-ken (JP); Matuo, Yuhsi, Suita-shi, Osaka-fu (JP)
(74) Representative: Tiedtke, Harro, Dipl.-Ing.

(57) **Abstract**

The concentration of phosphoglyceric acid mutase (PGAM) in a cell culture is assayed by a reagent containing enolase, pyruvate kinase, NADH, and lactate dehydrogenase. PGAM is useful as a damage marker of various cells or a cell toxicity marker thereof.

## Description

### DETAILED DESCRIPTION OF THE INVENTION

### TECHNICAL FIELD TO WHICH THE INVENTION BELONGS

The present invention relates to an assay system of cell damage and/or cell toxicity, and the invention can be utilized, for example, for determining or assaying cell damage due to ischemic disorders and the like.

### PRIOR ART

Phosphoglyceric acid mutase (sometimes referred to as PGAM: EC 5.4.2.1) is one of glycolytic enzymes and catalyzes the interconversion between 2-phosphoglycerate (Glycerate-2-P) and 3-phosphoglycerate (Glycerate-3-P) in the presence of 2, 3-bisphosphoglycerate (Glycerate-2,3-P2).

Mammalian animals carry two PGAM-encoding genes. The gene of the subunit M (molecular weight of about 30,000) is expressed in adult skeletal muscle and cardiac muscle, and in these tissues, homo-dimer MM (also referred to as type-M PGAM, M-PGAM, PGAM-MM, PGAM-M or type-M isozyme) is present. The gene of the subunit B (molecular weight of about 30,000) is expressed in adult brain, liver, kidney and erythrocyte, and in these tissues, homo-dimer BB (also referred to as type-B PGAM, B-PGAM, PGAM-BB, PGAM-B or type-B isozyme) is present. Accordingly, type-M PGAM is classified as muscle-specific isozyme and type-B PGAM is classified as non-muscle type or brain-type isozyme. The genes of both the subunits M and B are specifically expressed in cardiac muscle, and in the tissue, hetero-dimer MB (also referred to as type-MB PGAM, MB-PGAM, PGAM-MB or type-MB isozyme) is present, in addition to the type-B and type-M PGAMs.

By using isoelectric focusing with a potency to separately assay PGAM isozymes, Yates et al. revealed that the PGAM activity in normal plasma was derived from the type-B isozyme. Additionally, Markert demonstrated that the type-B PGAM isozyme was mainly present in the brains of normal human fetuses and adults; that the type-MB and type-M isozymes were observed in brain tumor tissues and the expression levels thereof had a correlation with the malignancy level of the tumor; and that such modification could not be observed in creatinine kinase (CK) as a muscle-specific enzyme. However, not so many research works have been made so far about the utilization of the PGAM and the isozymes thereof, so only the use of the type-B PGAM as a brain stroke marker (serum sample) has been reported in a patent application previously submitted by the present inventors (JP-A-8-322595). No report has been issued yet about the relation between the oxygen level at cell level and PGAM, as is established by the present invention. It is needless to say that the application of the PGAM and the isozymes thereof as markers of cell damage or cell toxicity has absolutely never been known.

### PROBLEMS THAT THE INVENTION IS TO SOLVE

It has been known that cell damage is triggered by a less oxygen state due to ischemia or the like or by the change of various conditions such as pH and temperature, and the development of a system for assaying cell damage or cell toxicity in an accurate and speedy manner has been needed greatly.

### MEANS FOR SOLVING THE PROBLEMS

The present invention has been attained for the purpose of satisfying the demand from the industry. As a consequence of research works in diverse fields, it has firstly been found that the PGAM value is escalated in a cell culture at a less oxygen state and that the less oxygen state can be assayed by the assay of not only total PGAM but also the isozymes thereof during PGAM assay, indicating the cellular damage level and additionally indicating a cellular damage level due to the change of various conditions. The inventors have got such novel and useful findings, and have attained the invention after additional research works.

More specifically, the inventors have firstly demonstrated that PGAMs (total PGAM, type-B PGAM, type-M PGAM) are useful as cell damage markers or cell toxicity markers, and have additionally verified that the assay of PGAMs in cells or cell culture permits the assay of cellular less oxygen state, the change of other conditions, and the extent of damage due to the change, and that the method previously developed by the inventors (JP-A-8-322595)is applicable as the method for assaying PGAM and the isozymes thereof. Thus, the invention has been achieved.

The invention will now be described in detail. When cells are damaged via the change of individual factors such as oxygen, pH, temperature and various chemical agents, these factors are generically referred to as cell toxicity in accordance with the invention. Hence, the cell damage marker serves indirectly as a cell toxicity marker, and the two markers are complimentary. In accordance with the invention, furthermore, the term less oxygen state means a state at an oxygen content lower than the oxygen content in atmosphere or a state with no oxygen.

The method for assaying PGAM in accordance with the invention will now be described about the case of the type-B isozyme. The method for assaying the type-B isozyme comprises treating a sample with a PGAM inhibitor to inactivate specific isozymes and assaying the remaining PGAM activity. When tetrathionic acid is used as the PGAM inhibitor, for example, almost 100 % of the type-M isozyme is inactivated; about 50 % of the type-MB isozyme is inactivated; and the type-B isozyme is hardly inactivated. By adding tetrathionic acid to serum to inactivate the type-M isozyme and subsequently assaying the residual PGAM activity, therefore, the type-B isozyme can be assayed separately.

At an inhibition experiment over purified PGAM isozymes, only 50 % of the type-MB isozyme is inactivated through the treatment with tetrathionic acid, so 50 % of the activity remains. Because the PGAM activity in normal serum and brain tissue is nearly totally derived from the type-B isozyme in a practical sense, the residual activity of the type-MB isozyme is negligible in biological samples.

As such PGAM inhibitor, use may be made of any substance capable of selectively inhibiting the activity of PGAM or isozymes thereof, including oxidants and SH reagents. Non-limiting examples thereof include polythionic acid and/or its derivatives. As polythionic acid, use maybe made of any one of trithionic acid to hexathionic acid; as the derivative thereof, use may be made of the potassium salt and sodium salt thereof and the like; and one of the preferable examples includes potassium tetrathionate.

The assay method of the invention comprises subjecting a sample to reaction with an inhibitor such as potassium tetrathionic acid to inactivate PGAM-M (the PGAM-MB activity is negligible for the present biological assay system as described above) and assaying the residual PGAM-B by using an assay reagent.

As described below in Table 1, the assay principle comprises inactivating the type-M isozyme by using potassium tetrathionic acid as the inhibitor of the type-M PGAM isozyme and assaying the activity of the PGAM-B as follows. More specifically, in the presence of 2,3-bisphosphoglyceric acid (Glycerate-2,3-P2), PGAM-B transforms 3-phosphoglyceric acid (Glycerate-3-P) into 2-phosphoglyceric acid (Glycerate-2-P), which is then modified into PEP and H₂O with enolase. In the presence of ADP, PEP is then decomposed into pyruvate and ATP with pyruvate kinase (PK), and the resulting pyruvate is subjected to reaction with lactate dehydrogenase (LDH) in the presence of NADH. The NADH reduction is rate assayed.

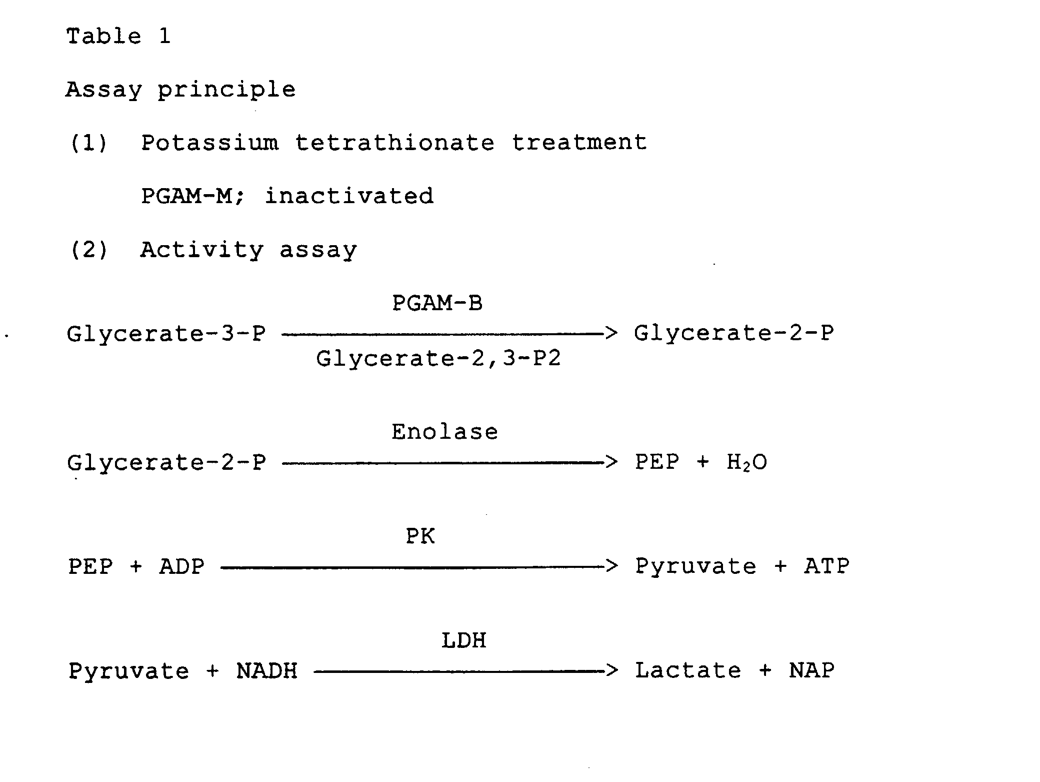

The NADH reduction can be assayed simply by means of commercially available assay systems, so that PGAM-B can accurately and speedily be assayed (this is true with the case of total PGAM) . As apparently shown in the following examples, the PGAM value in a cell culture sample at a less oxygen state is higher than the value in a cell culture sample at a more oxygen state, and such phenomenon is frequently observed in various cells. It is indicated that by simply selecting one readily assayable PGAM type (total PGAM, type-B PGAM, type-M PGAM or other isozymes) in each cell and assaying the value thereof, the cellular less oxygen state (ischemia, etc.) can be assayed by the assay of the PGAM. It is demonstrated that PGAM is extremely preferable as a cell damage marker, particularly a damage marker of cells affected by ischemic disorders or damages with less oxygen. Conventionally, these findings have never been known. It is thus confirmed that PGAM is a novel cell damage marker. In accordance with the invention, additionally, cellular damages due to various cell toxicities other than less oxygen can be assayed, and therefore, the marker may also be used as cell damage markers applicable to an extremely wide range; in other words, the marker may be applicable as a cell toxicity marker. The invention furthermore provides a PGAM-B assay reagent including enolase, pyruvate kinase (PK), NADH, lactate dehydrogenase (LDH) and a PGAM inhibitor and including a substrate and a buffer solution if necessary, and the reagent can be utilized not only as a reagent for assaying a less oxygen state but also as a reagent for assaying various cell toxicities other than less oxygen. As a kit of reagent 1 and reagent 2, the reagent may be introduced into market, as shown in the following examples.

The assay of PGAM-B has been described above, and total PGAM (sometimes simply referred to as PGAM or T-PGAM) also exerts the same performance profile as that of the type-B isozyme; in absolutely the same manner, T-PGAM and PGAM-M can be utilized for assaying a less oxygen state or for determining other physical, chemical and biological factors with adverse effects on cells (ie. for determining cell toxicity) and can be utilized as a marker of cell damage and/or cell toxicity.

The assay method of T-PGAM is totally the same as the method of the type-B isozyme, except for no use of any inhibitor (the assay principle is totally the same, except for (1) the use of inhibitors and (2) the substitution of PGAM-B with T-PGAM), so the NADH reduction is simply assayed by the rate assay method. In a similar manner to the assay of the type-B isozyme, the assay of T-PGAM may be utilized for assaying a cellular less oxygen state or for assaying other cellular toxicities. In that case, the assay may satisfactorily be carried out with no use of any inhibitor. Furthermore, the value of the type-M isozyme can be yielded by reducing the B-PGAM value from the T-PGAM value.

An assay reagent of T-PGAM and a kit thereof are totally the same as those of the type-B isozyme, expect for the use of inhibitors, and based on the same reason as the reason described above, these may also be utilized very effectively as an assay reagent of less oxygen states and cell damage (toxicity) . The same is true with the assay reagent and kit of M-PGAM.

So as to practice the invention, PGAM in a cell culture and/or a cell debris or a solution isolated therefrom is assayed according to the methods, for example, as follows.

### (Culturing of smooth muscle cell)

From the thoracic aorta of a male SHRSP of age 8 to 10 weeks are collected smooth muscle cells by the explant method, and the cells are cultured in a DMEM culture medium containing 10 % fetal bovine serum (FBS).

### (Culturing of astrocyte)

From the brain of an SHRSP rat fetus of age 15 days are collected astrocytes for culture by the trypsin digestion method, and the astrocytes are then cultured in a DMEM culture medium containing 10 % FBS.

### (Less oxygen experiment)

These cells are cultured in 0.01 to 1 %, preferably 0.1 % FBS for about 12 to 60 hours (the duration varies depending on the cell, but is generally 48 hours), and are subsequently cultured in atmospheres at various oxygen concentrations (for example, at 20 % oxygen or 1 % oxygen) for given periods (for example, 2, 4 and 8 days). Then, the culture media are recovered. PGAM in these culture media is assayed. In that case, the same type of PGAM may satisfactorily be assayed in any of the culture media, but for a culture medium of a cell derived from brain, such as astrocyte culture medium, the type-B PGAM is assayed. PGAM and PGAM isozyme types suitable for individual cells may satisfactorily be selected.

Examples of the invention will be described hereinbelow.

### Example 1

From the thoracic aorta of a male SHRSP of age 8 to 10 weeks were collected smooth muscle cells by the explant method, and the cells were cultured to prepare cultured smooth muscle cells.

### (Explant procedures)

(1) While setting autopsy tools inside a clean bench, PBS was divided into 60-mm petri dishes.
(2) After anesthetizing a rat with pentobarbital, the abdominal hair was cut with a razor, and then, the rat was placed at a dorsal position on a TMS sheet.
(3) After drawing blood from the abdominal aorta (with a syringe), the aorta was resected above the diaphragm toward the aortic arc by opening the thoracic cavity and was then transferred in a 60-mm petri dish containing PBS.

### (Procedures inside clean bench)

(4) After removing loose connective tissues from the outer membrane of the aorta, the aorta was cut longitudinally with a pair of small scissors.
(5) After washing off blood attached to the tissue, the tissue was transferred in a 15-ml test tube containing 5 ml of a 1 mg/ml collagenase solution for subsequent incubation in a CO₂ incubator for 20 minutes (25 minutes for SP and SR and about 35 minutes for WKY).
(6) After completion of the incubation, the aorta was transferred in a PBS-containing petri dish and was washed gently therein. Transferring the aorta onto the lid of the petri dish, the aorta was opened while the inner cavity was held upward. By gently rubbing the inner membrane face with a cotton swab immersed in PBS, the residual endothelium was removed. Lightly cutting one end with a scalpel, the endomedia was peeled off from the outer membrane by means of a clock clamp (the procedure was required to be done speedily not to dry the tissue).
(7) The media tissue was cut into small pieces of about 1-mm square. (The procedure was also required to be done speedily not to dry the tissue.)
(8) Each tissue piece was picked up with a 26-G needle onto the tip of the cotton swab and was then attached to the bottom of a 25-cm² flask. During the procedure, about 30 tissue pieces were attached to one flask (the procedure was speedily conducted.)
(9) While avoiding that the DMEM-F (+) culture medium might be poured on the tissue, the culture medium was added to the flask.
(10) The flask was sealed with a stopper and was left to stand inside a CO₂ incubator so as to allow the adhesion of the tissue pieces to the flask face. After the adhesion of the tissue pieces (10 to 20 minutes later; when the PBS around the periphery of the tissue pieces was less), the flask was slowly laid on its side to permit the tissue pieces to be immersed in the culture medium (attention was paid so as not to dry the tissue pieces).
(11) After leaving the flask for 5-7 days to stand alone, the migration state was observed. The culture medium in which the migration was observed was replaced with fresh one of the medium, and then, the flask was further left to stand.
(12) After sufficient volumes of cells migrated and grew, the cells were subjected to passage (the tissue pieces should be removed as much as possible, but it is permitted that the tissue pieces might remain).

### Example 2

After culturing the resulting cultured cells (cells after passage from 5 generations to 10 generations) prepared in Example 1 in 0.1 % FBS for 48 hours, the resulting cells were cultured in an atmosphere at 20 % oxygen or 1 % oxygen for 2, 4 and 8 days, to recover individual culture media which were designated as samples. These samples (n = 6 for each group) were subjected to the assay of total PGAM (T-PGAM) with an automatic analyzer of Hitachi 7150 according to the procedures and conditions shown below in Table 2.

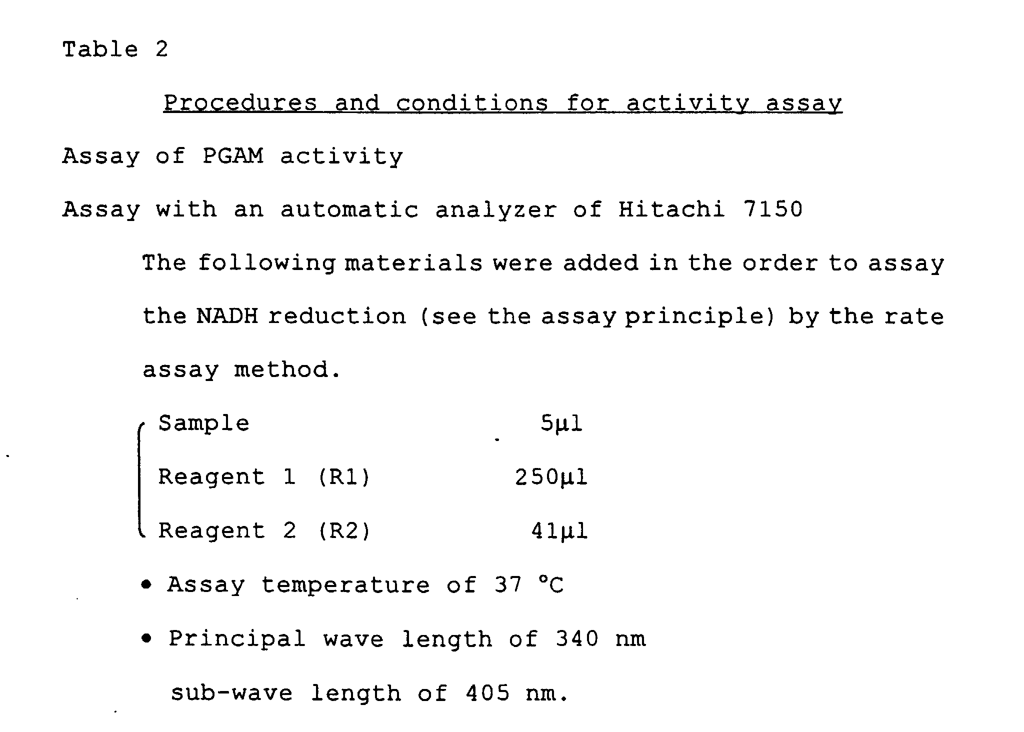

As the assay reagents, reagent 1 (R-1) and reagent 2 (R-2) with the following compositions were used.

| (Reagent 1) : R-1 | | |
|---|---|---|
| TEA buffer | (0.1 mol/liter, pH 7.6) | 40.32 ml |
| MgSO₄ | (0.1 mol/liter) | 0.54 |
| NADH | (14 mmol/liter) | 0.90 |
| ADP | (21 mmol/liter) | 1.80 |
| G-2, 3-P2 | (7 mmol/liter) | 0.90 |
| LDH | (5 mg protein/ml) | 0.18 |
| PK | (2 mg protein/ml) | 0.18 |
| enolase | (10 mg protein/ml) | 0.18 |
| | | (total: 45.00 ml) |

| (Reagent 2) : R-2 | | |
|---|---|---|
| G-3-P | (95 mmol/liter) | 3.00 ml |
| TEA buffer | (0.1 mol/liter, pH 7.6) | 5.20 |
| | | (total: 8.20 ml) |

The reagent 1 (R-1; 250 µl) was mixed with a culture medium sample (5 µl), and the resulting mixture was then left to stand for 5 minutes. By subsequently using the reagent 2 (R-2), the PGAM activity was assayed.

Because the reagent 2 (R-2) contained glycerate-3-P (substrate), the reaction was initiated by adding 41 µl of the reagent 2. About 1.5 minutes after the addition of the reagent 2, the assay was started.

By using an automatic analyzer of Hitachi 7150 and by presetting the assay code to RATE-A: 32-39 and the assay temperature to 37 °C, the decrease of absorbance A at 340 nm was determined on the basis of the reduction of NADH for about 1.5 minutes. Additionally, a sub-wave length was set at 405 nm for the determination. The results are shown below in Table 3. Furthermore, the value of a conventionally known cell damage marker LDH (lactate dehydrogenase) is shown concurrently.

**Table 3**

| T-PGAM | | | |
|---|---|---|---|
| | 2 Days later | 4 Days later | 8 Days later |
| 20 % O₂ | 7.9 | 6.6 | 7.0 |
| 1 % O₂ | 9.5 | 16.7 | 85.2 |
| P | ns | <0.0001 | <0.000001 |

| LDH | | | |
|---|---|---|---|
| | 2 Days later | 4 Days later | 8 Days later |
| 20 % O₂ | 10.2 | 8.9 | 7.5 |
| 1 % O₂ | 6.1 | 10.2 | 106.8 |
| P | ns | ns | <0.000001 |

As apparently indicated from the results of the cell damage tests under exposure to less oxygen by using the SHRSP-derived smooth muscle cells, total PGAM value (U/L/mg protein) under exposure to less oxygen was higher than the value under exposure to more oxygen, which suggests that PGAM is applicable as a marker of cell damage. It is verified that PGAM is a more sensitive marker rather than LDH.

### Example 3

From the brain of an SHRSP rat fetus of age 15 days were collected astrocytes for culture by the trypsin digestion method, and the astrocytes were then cultured and prepared as cultured astrocytes.

### (With alcohol disinfection)

(1) Brain was resected from a rat fetus of age 15 to 22 days.
(2) The brain was placed in PBS(-) in a petri dish.

### (Treatment inside clean bench)

(3) Dura was peeled off. The brain was cleaned and cut into fine pieces.
(4) Approximately three times, the petri dish was exchanged to a fresh petri dish at the process of cutting the brain into fine pieces, to prepare clean pieces.
(5) Antibiotics (streptomycin and penicillin) were added at a concentrate about 10-fold the concentrate thereof for general use of cell culture to the pieces, which were then left to stand for 5 minutes (two times).
(6) The fine pieces were treated with 0.25 % trypsin (37 °C, for 5 to 7 minutes).
(7) By using a Pasteur pipette, the pieces were subjected to pipetting (10 to 20 times) at such an extent that the tissue pieces might be disrupted.
(8) The pieces were centrifuged at 12,000 rpm for 5 minutes, to recover a pellet.
(9) DMEM + 10 % FCS were added to the pellet, to terminate the trypsin reaction.
(10) The resulting material was centrifuged at 12,000 rpm for 5 minutes, to recover a pellet.
(11) One brain was cultured in 6 ml of the culture medium in a 25-cm² flask.

### Example 4

Prior to the assay of B-PGAM in the cell culture medium, the effect of potassium tetrathionate on PGAM-B and PGAM-M was examined by the automatic analysis method. As the sample, a type-B standard and a type-M standard (both based on pooled sera) were used. These standards were diluted with physiological saline to prepare 1/1 to 1/8 samples. Various concentrations of potassium tetrathionate were added to these samples for 5-min reaction, and subsequently, the amounts of the type-B and type-M PGAMs were determined by utilizing the automatic analysis method.

By using the reagents of Example 2, the amounts of PGAM-B and PGAM-M were individually determined by the automatic analysis method, and the results are shown below in Table 4. The results apparently reveal that only the type-M isozyme is specifically inactivated with potassium tetrathionate but the type-B isozyme is absolutely never influenced.

**Table 4**

| Effect of potassium tetrathionate over PGAM-B and PGAM-M | | | | | |
|---|---|---|---|---|---|
| | dilution ratio | Final potassium tetrathionate concentration (mM) | | | |
| | | 0 | 1.9 | 3.8 | 7.6 |
| PGAM-B standard (based on pooled sera) | 1/8 | 100( 22U/L) | 93 | 93 | 75 |
| | 1/4 | 100( 46U/L) | 97 | 86 | 59 |
| | 1/2 | 100( 91U/L) | 98 | 91 | 80 |
| | 1/1 | 100( 183U/L) | 98 | 92 | 81 |
| PGAM-M standard (based on pooled sera) | 1/8 | 100( 322U/L) | 0 | 0 | 0 |
| | 1/4 | 100( 676U/L) | 0 | 0 | 0 |
| | 1/2 | 100(1365U/L) | 0 | 0 | 0 |
| | 1/1 | 100(2727U/L) | 0 | 0 | 0 |
| unit; % | | | | | |

### Example 5

The cultured cell (cell after passage over 5 to 10 generations) prepared in Example 3 was cultured in 0.1 % FBS for 48 hours and was then cultured in an atmosphere at 20 % oxygen or 1 % oxygen for 2, 4 and 8 days, to recover individual culture broths, which were designated as samples.

The PGAM activity of the samples was assayed by using potassium tetrathionate as an inhibitor of the type-M PGAM isozyme. As an assaying reagent, 2.25 mM potassium tetrathionate (molecular weight of 302.4) (to a final concentration of 1.9 mM) was added to the PGAM activity-assaying reagent (R-1) used in Example 1 [potassium tetrathionate (15 mg) was added to 22 ml of the reagent (R-1)], for the inactivation of the type M, to prepare a type-B PGAM activity-assaying reagent.

250 µl of the reagent 1 (R-1) with addition and dissolution of potassium tetrathionate therein and 5 µl of a sample were mixed together, and the resulting mixture was left to stand for 5 minutes. Because the type-M PGAM (and type MB) in the sample was inactivated and blocked during the term, the type-B PGAM value (U/L/mg protein) was subsequently assayed by using the reagent 2 (R-2). The results are shown in Table 5.

**Table 5**

| B-PGAM | | |
|---|---|---|
| | 2 Days later | 8 Days later |
| 20 % O₂ | 12.20 ± 0.57 (n=3) | 10.72 ± 0.70 (n=3) |
| 1 % O₂ | 15.26 ± 0.54 (n=3) | 30.11 ± 2.94 (n=3)...P<0.01 |
| mean SE, p<0.01 from 20 % O₂ | | |

As apparently shown in the results of the cell damage tests under exposure to less oxygen by using the SHRSP-derived cultured astrocytes, the PGAM value under exposure to less oxygen is higher than the value under exposure to more oxygen. Hence, it is indicated that B-PGAM can also be used as a marker for cell damage. Furthermore, attempts were made to assay LDH, but the value thereof was too low. Therefore, LDH could not be used as such marker.

### EFFECTS OF THE INVENTION

By assaying PGAM (including T-PGAM, B-PGAM and other isozymes) in accordance with the invention, less oxygen state can be assayed, whereby cell damage, particularly damages with less oxygen such as ischemic disorders, can be assayed. Additionally, PGAM can be used widely as a damage marker of various cells. In accordance with the invention, furthermore, cell damages due to various cell toxicities except for less oxygen can be widely assayed. Therefore, PGAM can be used as a marker of diverse cell damages (a cell toxicity marker from another standpoint).

The concentration of phosphoglyceric acid mutase (PGAM) in a cell culture is assayed by a reagent containing enolase, pyruvate kinase, NADH, and lactate dehydrogenase. PGAM is useful as a damage marker of various cells or a cell toxicity marker thereof.

## Claims

1. A reagent for assaying cell damage and/or cell toxicity, comprising an assay reagent of phosphoglyceric acid mutase (PGAM) or an isozyme thereof.

2. A reagent according to claim 1, wherein the isozyme of PGAM is type-B isozyme or type-M isozyme.

3. A reagent according to claim 1 or 2, wherein the assay reagent of PGAM contains enolase, pyruvate kinase, NADH, and lactate dehydrogenase.

4. A reagent according to any one of claims 1 to 3, wherein the assay reagent of the type-B isozyme contains a PGAM inhibitor.

5. A reagent according to claim 4, wherein the PGAM inhibitor is polythionic acid and/or a derivative thereof.

6. A reagent according to claim 5, wherein the PGAM inhibitor is the potassium salt or sodium salt of tetrathionic acid.

7. A method for assaying cell damage and/or cell toxicity, comprising assaying PGAM in a sample by using a reagent according to any one of claims 1 to 3 by the rate assay method.

8. A method for assaying cell damage and/or cell toxicity, comprising treating a material by using a PGAM inhibitor and subsequently assaying a PGAM isozyme by the rate assay method.

9. A marker of cell damage or cell toxicity, comprising PGAM or an isozyme thereof.

10. A marker of cell damage or cell toxicity according to claim 9, wherein the cell damage is caused by oxygen.
